# NEW EUROPEAN PATENT SPECIFICATION

(11) **EP 1 105 495 B2**
(45) Date of publication and mention of the opposition decision: **21.01.2009**
(45) Mention of the grant of the patent: 16.03.2005
(21) Application number: 99940986.5
(22) Date of filing: 13.08.1999
(51) Int. Cl.: C12N 15/37, C07K 14/205, A61K 39/12

(54) **METHOD FOR PRODUCING YEAST EXPRESSED HPV TYPES 6 AND 16 CAPSID PROTEINS**
VERFAHREN ZUR HERSTELLUNG VON DURCH HEFE EXPRIMIERTE HPV TYPS 6 UND 16 KAPSID-PROTEINEN
PROCEDE POUR PRODUIRE DES PROTEINES DE CAPSIDES DE TYPES 6 ET 16 DU PVH EXPRIMEES DANS DES LEVURES

(30) Priority: 14.08.1998 US 96625 P
(43) Date of publication of application: 13.06.2001
(73) Proprietor: Novartis Vaccines and Diagnostics, Inc., Emeryville, CA 94608 (US)
(72) Inventor: BUONAMASSA, Daniela, Tornese, Chiron SpA, 53100 Siena (IT); GREER, Catherine, E., Chiron Corporation, Emeryville, CA 94608 (US); GALEOTTI, Cesira, L., Chiron SpA, 53100 Siena (IT); BENSI, Giuliano, Chiron SpA, 53100 Siena (IT); PETRACCA, Roberto, Chiron SpA, 53100 Siena (IT)
(74) Representative: Hallybone, Huw George
(86) International application number: PCT/US1999/018016
(87) International publication number: WO 2000/009699

(56) References cited:
- WO-A-96/05293
- WO-A-98/14564
- CHANG C ET AL: "Phenotypic mixing between different hepadnavirus nucleocapsid proteins reveals C protein dimerization to be cis preferential" JOURNAL OF VIROLOGY, vol. 68, no. 8, August 1994 (1994-08), pages 5225-5231, XP002130379 AMERICAN SOCIETY FOR MICROBIOLOGY US cited in the application
- HOFMANN K J ET AL: "Sequence conservation within the major capsid protein of human papillomavirus (HPV) type 18 and formation of HPV-18 virus-like particles in Saccharomyces cerevisiae" JOURNAL OF GENERAL VIROLOGY,GB,SOCIETY FOR GENERAL MICROBIOLOGY, READING, vol. 77, no. 3, March 1996 (1996-03), pages 465-468, XP000604634 ISSN: 0022-1317

## Description

The present application claims priority under 35 U.S.C. § 119(e) to Provisional Application Serial No. 60/096,625, filed August 14,1;998, said . application incorporated by reference herein in its entirety.

### Field of the Invention

The present invention is related to the production of mosaic virus-like particles comprising capsid proteins of human papilloma virus (HPV) types 6 and 16 capable of inducing immune response against both HPV types.

### Background of the Invention

A promising strategy to induce an immune response capable of neutralizing papillomavirus (PV) infections is the use of virus capsid proteins as antigens. In the case of genital human papillomaviruses (HPVs), this approach was hampered by the lack of any *in vivo* or *in vitro* source of sufficient amounts of native virus. In order to overcome this problem, heterologous expression systems have been extensively used to obtain large quantities of capsid proteins and to allow the analysis of their structural and immunological properties. Expression of the major capsid protein late 1 (L1) from different PV types using prokaryotic (25), baculovirus (21, 23, 37, 41, 42, 46), yeast (14, 18, 19, 20, 29) and mammalian expression systems (15, 16, 51), demonstrated that this protein can self-assemble into virus-like particles (VLPs). Coexpression of the minor capsid protein late 2 (L2) is not strictly necessary to obtain VLPs, although its presence increases the efficiency of particle formation (15, 22, 51) and induces anti-L2 neutralizing antibodies (32). The L1 and L2 VLPs appear similar to native vinous by electron microscopy (EM). The use of different animal models has shown that VLPs can be very efficient at inducing a protective immune response.

VLPs meet many of the criteria which make them ideal surrogates of native virions. They resemble infectious particles by ultrastructural analysis (16), elicit virus neutralizing antibodies and bind to the putative receptor on the surface of mammalian cells (28, 31, 33, 44, 47). Most notably, the results obtained with animal models demonstrated that prophylactic immunization with VLPs can be very effective *in vivo*. Cottontail rabbits, calves and dogs immunized with L1 VLPs were protected from subsequent challenge with the homologous PV (20, 23, 41) and passive transfer of immune sera conferred protection to naive animals (20, 41), indicating that an antibody-mediated response plays a major role in preventing virus infection.

Studies with infectious HPV virions, as well as VLPs of different HPV types, strongly suggested, however, that the immune response is predominantly type-specific. Further, the efficacy of VLP-based anti-HPV vaccine candidates cannot be evaluated in animals since these viruses exhibit a high degree of species specificity. Antibody-mediated virus neutralization has been therefore studied using either *in vitro* assays (35, 40) or xenograft systems which allow propagation of infectious virus of specific HPV types (1, 2, 5, 6, 24). The primary conclusion which could be drawn from these experiments was that immunization with HPV VLPs evokes a neutralizing immune response which is predominantly type-specific (6, 7, 34, 35, 36, 48).

Cross-neutralization has been reported between HPV-6 and HPV-11 (92% amino acid sequence identity) (8) and between HPV-16 and HPV-33 (80% amino acid sequence identity) (48). This may indicate the existence of some correlation between protein sequences and structural similarities that could possibly be relevant for the mechanism of capsid assembly. On the basis of these considerations, however, the concept that HPV-6 and HPV-16 L1 proteins may coassemble is not obvious, since the two viruses belong to phylogenetically more distant groups (3, 45) and exhibit a lower (67%) L1 amino acid sequence identity.

Further, while envelope proteins of viruses belonging to very different families can be incorporated into the same envelope (50), nucleocapsid protein mixing appears to be much more restricted. Mixed core particles between Moloney murine leukaemia virus (MuLV) and human immunodeficiency virus (HIV) have been obtained but only when artificial chimeric Gag precursors, containing both HIV and MuLV determinants are coexpressed with wild-type MuLV Gag proteins (10). By using a yeast two-hybrid system based on GAL4-Gag fusion protein expression plasmids, Franke et al. were able to show that the ability of two heterologous Gag proteins to multimerize was correlated with the genetic relatedness between them (13).

Mixed capsid formation between wild-type Gag proteins has not been reported so far. In the case of the hepadnavirus core (C) protein, Chang et al. (4) have shown that an epitope-tagged truncated hepatitis B virus (HBV) C polypeptide could coassemble in Xenopus oocytes with woodchuck hepatitis virus (WHV) and ground squirrel hepatitis virus (GSHV) C proteins but not with that of duck hepatitis B virus (DHBV). This result was not unexpected since the two core protein sequences have diverged significantly and do not show immunological cross-reactivity. When coassembly of C polypeptides of HBV, WHV and GSHV occurred, formation of mixed capsids resulted from the aggregation of different species of homodimers (4). Synthetic HPV16 VLPs have been described that have been mutated in certain regions so that HPV11 specific antibodies will bind (53). Flock House Virus particles have also been described where heterologous amino acid sequences comprising, for example, epitopes have been inserted into the capsid protein (54).

Several reports have discussed the importance of disulfide bonds for the integrity of native bovine papillomavirus type 1 (BPV-1) virions (26) and VLP structures (25,38, 39). Li et al. (26) have also shown that the cysteine 424 mutant (C424) of HPV-11 L1 in the carboxy-terminal domain that has been identified as critical for capsid formation (25), is still able to form capsomeres but not VLPs, indicating that this residue may be involved in interpentamer bonding. The essential role of disulfide bonds has been confirmed by a single point mutation of either C 176 or C427 in HPV-33 L1 (C428 in HPV-18 Ll), which converts all VLP trimers into monomers, allowing capsomere formation but not VLP assembly (39).

It has been recently proved that, by using an in vitro infection system and a sensitive reverse transcriptase PCR-based assay (RT-PCR), antisera to HPV-6 VLPs are not able to neutralize authentic HPV-16 virions (48). Since cysteine residues corresponding to those described as involved in disulfide bonding above are conserved in the HPV-6 and HPV-16 LI proteins, we hypothesized that mosaic VLPs could either result from intra-capsomeric or inter-capsomeric association of the two proteins and/or from interaction between type-specific subsets of capsomeres.

### Summary of the Invention

In one aspect, the present invention relates to a method for producing mosaic virus like particles comprising the capsid proteins from HPV types 6 and 16. In a futher preferred aspect, the capsid proteins are L1 and L2 from HPV types 6 and 16.

In a further aspect, the present invention relates to vectors and hosts for expressing the capsid proteins of at least two types of HPV. In a preferred aspect, the capsid proteins are from HPV types 6 and 16. In a futher preferred aspect, the capsid proteins are L1 and L2 Rom HPV types 6 and 16. In a further preferred aspect, the present invention relates to a diploid yeast strain that coexpresses the L1 and L2 capsid proteins of HPV-6 and HPV-16 as mosaic VLPs.

In another aspect, the present invention relates to the use of VLPs comprising capsid proteins from HPV types 6 and 16 for inducing an immune response. In a futher preferred aspect, the mosaic VLPs comprise the L1 and L2 capsid proteins from HPV types 6 and 16.

In still another aspect, the present invention relates to an immunogenic virus like particle comprising capsid proteins from different types of HPV, most preferably HPV types 6 and 16. In a futher preferred aspect, the mosaic VLPs comprise the L1 and L2 capsid proteins from HPV types 6 and 16.

### Brief Description of the Drawings

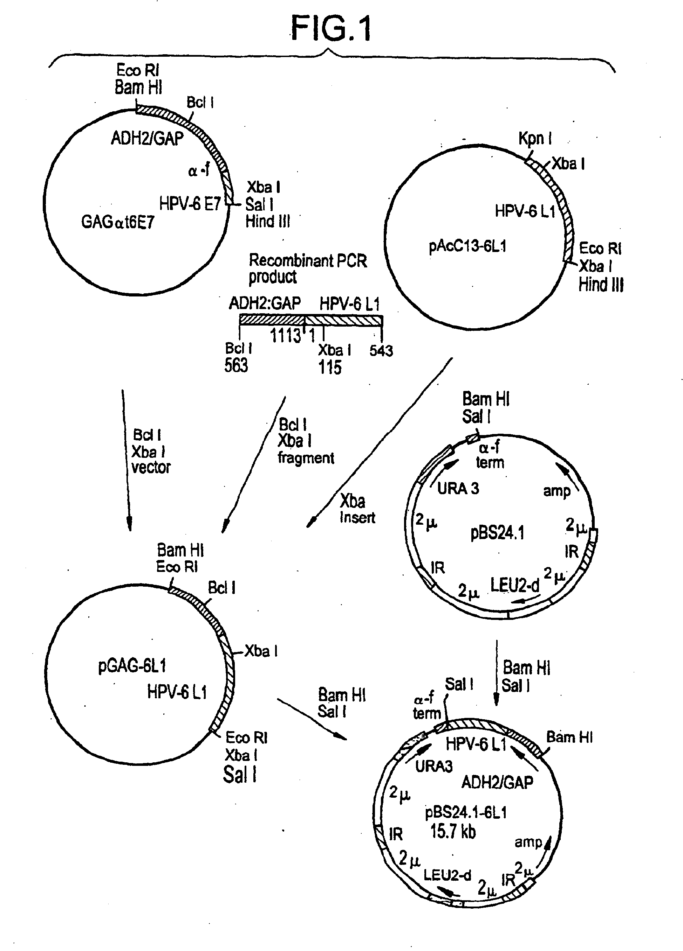

FIG. 1 is a schematic of the construction of the pBS-6L1 plasmid.

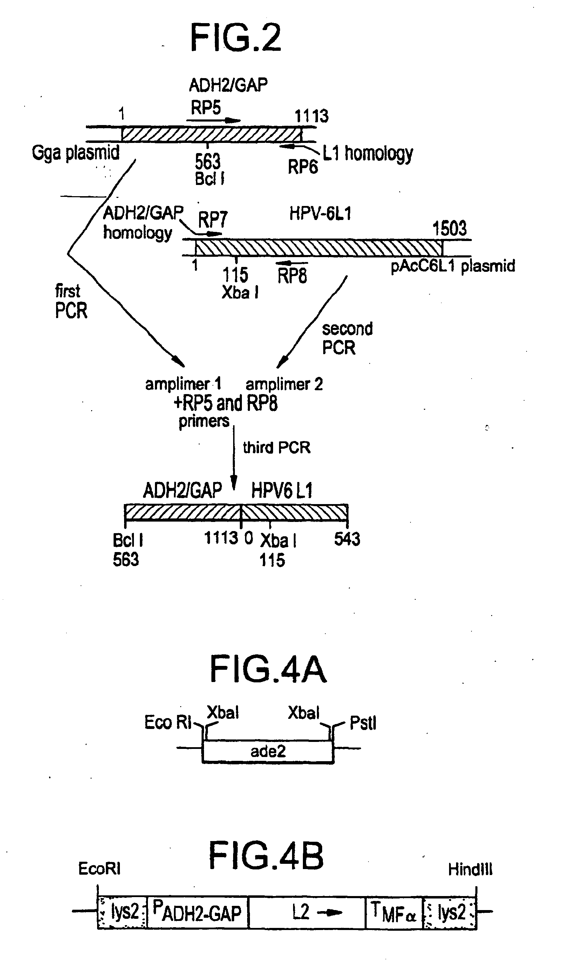

FIG. 2 depicts the recombinant PCR performed in constructing the pBS-6L1 plasmid.

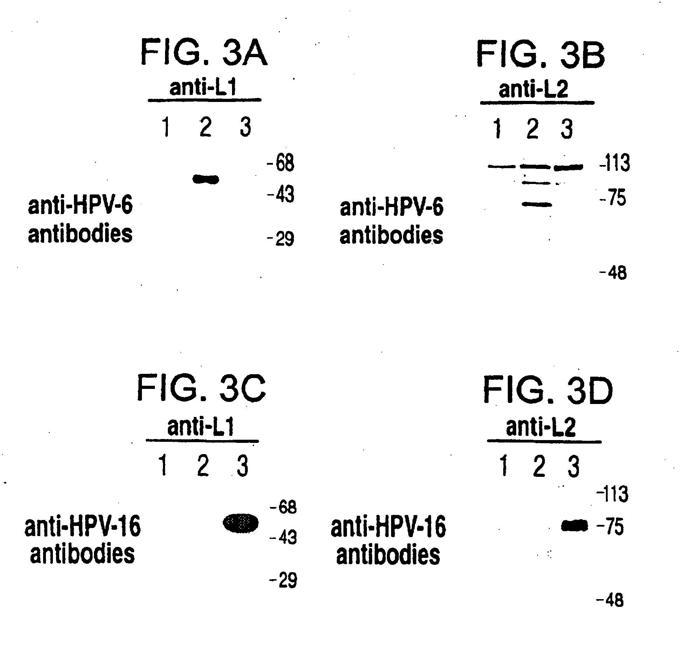

FIG. 3 depicts a Western blot analysis of cell extracts from yeast strains expressing HPV-6 and HPV-16 capsid proteins. Equivalent amounts of total cell extracts from the parental JSC310 strain (lanes 1) and different recombinant strains (lanes 2 and 3) were separated by 10% SDS-PAGE, electrotransferred to nitrocellulose filters and incubated with the H6.C6 (a) or the H16.H5 (c) type-specific anti-L1 Mabs, and with HPV-6L2 (b) or HPV 16L2 (d) antisera. Lanes 2a and 2c: JSC310-6Llepi; lanes 3a and 3c: JSC310-16L1epi; lanes 2b and 2d: JSC310-6L2epi; lanes 3b and 3d: JSC310-16L2epi. Molecular mass standards (in kDa) are indicated. This multipanel figure and those which follow have been assembled by using Photoshop 4.0 and FreeHand 7.0 programs for Macintosh.
FIG. 4 is a schematic representation of the yeast integrative plasmids YIpAde (a) and YIpLys-L2 (b) vectors. The continuous lines represent pUC vector sequences. The box labelled ade 2 in (a) represents the adenine 2 gene sequence. The boxes labelled lys 2 in (b) represent lysine 2 gene fragments, the box labelled L2 represents the L2 gene, the other boxes represent the ADH2/GAP hybrid promoter and the MFα gene transcriptional termination sequence. The arrow in the L2 box indicates the 5'-3' orientation of the coding sequence. Relevant restriction sites are indicated.

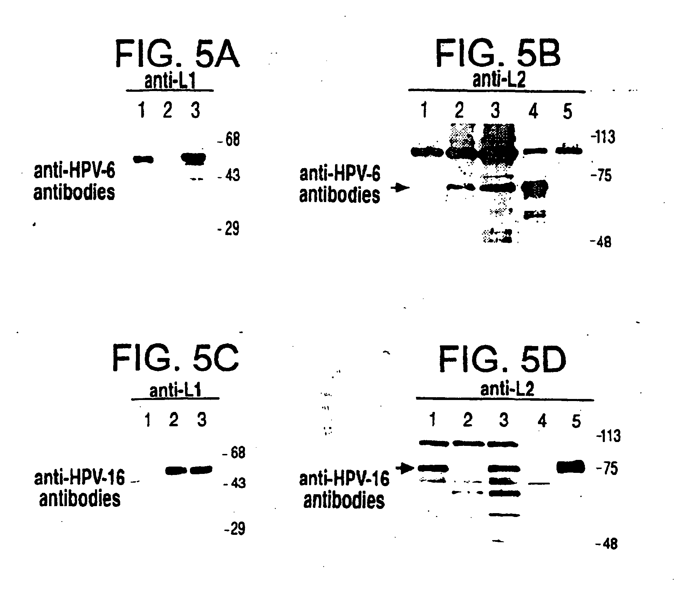

FIG. 5 depicts a Western blot analysis of cellular extracts from recombinant haploid and diploid yeast strains. Total cell extracts were separated by 10% SDS-PAGE, electrotransfened to nitrocellulose filters and incubated with anti-HPV-6 L1 (a) and anti HPY 16 L1 (c) Mabs and with HPV-6 L2 (b) and HPV-16 L2 (d) antisera. Lanes 1: AB110-6Lt/16L2; lanes 2: JSC310-16L1/6L2; lanes 3: AB/JS-4L; lanes 4: JSC310-6L2epi; lanes 5: JSC310-16L2epi. Arrows in (b) and (d) indicate the bands corresponding to the L2 proteins. Molecular mass standards (in kDa) are indicated.

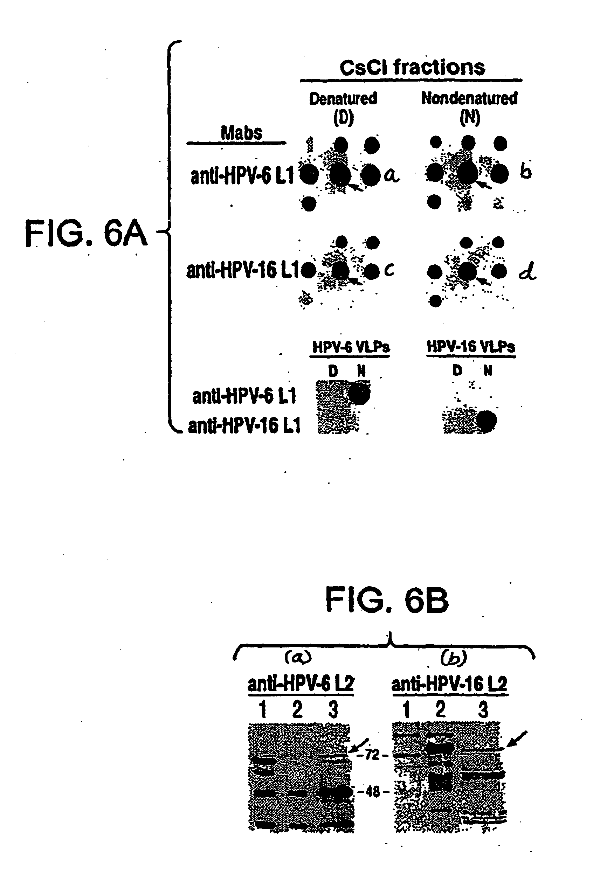

FIG. 6 depicts an analysis of fractions from CsCl gradient sedimentation of AB/JS-4L cell extract. (A) Aliquots from factions 1 to 9 were blotted onto nitrocellulose filters using either (a and c) denaturing and reducing (D) or (b and d) nondenaturing and nonreducing (N) conditions. The filters were incubated with the type-specific anti-L1 H6.C6 (a) and H16.H5 (c) Mabs, and with the conformationally dependent type-specific anti-L1 H6.B10.5 (b) and H16.V5 (d) Mabs. As a control, the anti-HPV-6 and HPV-16 L1 conformational Mabs were incubated with CsCl purified VLPs (e) blotted under either denaturing or nondenaturing conditions. The arrows in A indicate fraction no. 5. (B) Aliquots of fraction no. 5 were subjected to SDS-PAGE, electroblotted on nitrocellulose filters and incubated either with HPV-6 L2 (lane 3a) or HPV-16 L2 (lane 3b) antiserum. As a control, total cell extracts from the JSC310-6L2epi (lanes 1) and JSC310-16L2epi (lanes 2) strains were used. Molecular mass standards (in kDa) are indicated. Arrows indicate bands corresponding to the L2 proteins.

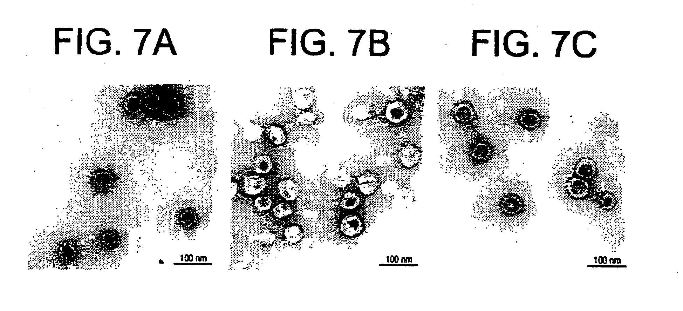

FIG. 7 depicts an electron microscope (EM) analysis of CsCl purified VLPs. HPV-6 (a), HPV-16 (b) and IHPV-6/16 VLPs were adsorbed onto Formvar-carbon coated grids, stained with 4% uranyl acetate and examined under a Zeiss EM10C microscope at a magnification of x 100,000 (Bar=100nm).

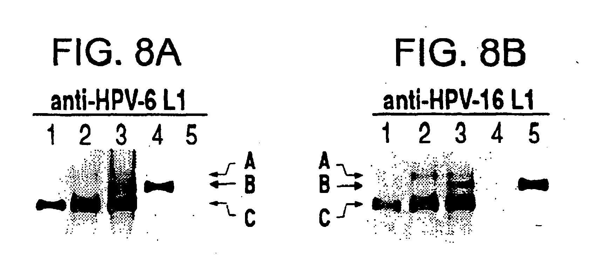

FIG. 8 depicts a Western blot analysis of immunoprecipitated VLPs. CsCl banded VLPs from the AB/JS-4L diploid strain were immunoprecipitated with the anti-HPV-6 L1 conformationally dependent H6.B 10.5 Mab. The immunoprecipitated proteins were separated using a 15 centimeter (cm) long 10% polyacrylamide SDS-gel, electroblotted on nitrocellulose membrane and incubated either with the anti-HPV-6 L1 specific H6.C6 Mab (a) or with the anti-HPV-16 L1 specific H16.H5 Mab (b). Control reactions, including either VLPs or the conformational Mab only, were set up and processed under identical experimental conditions. Lane 1: VLPs incubated overnight without the Mab; lane 2: Mab incubated overnight; lane 3: VLPs incubated overnight with the H6.B 10.5 conformational Mab; lane 4: total cell extract from the JSC310-6L1epi strain; lane 5: total cell extract from the JSC310-16L1epi strain. Arrows indicate a conformational Mab-derived band (A), the L1 bands (B) and a protein A Sepharose-derived band (C).

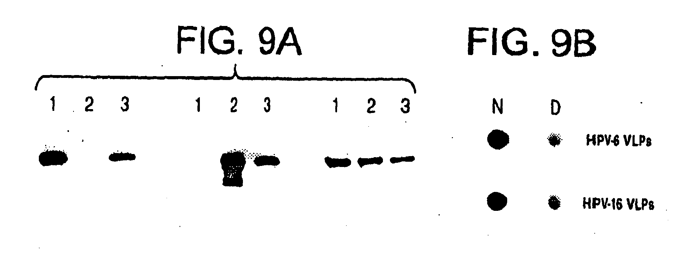

FIG. 9 depicts a characterization of sera derived from mice immunized with HPV-6, HPV-16 and mosaic VLPs. (A) Comparable amounts of HPV-6 (lanes 1), HPV-16 (lanes 2) and mosaic VLPs (lanes 3) were separated on SDS-PAGE and immunoblotted with antisera from mice immunized with HPV-6 VLPs (a) HPV-16 VLPs (b) and mosaic VLPs (c). (B) Comparable amounts of HPV-6 and HPV-16 VLPs were dot-blotted under denaturing and reducing (D) and nondenaturing and nonreducing (N) conditions and incubated with the S 16 antiserum of a mice immunized with mosaic VLPs.

### Detailed Description of the Invention

To test the possibility of inducing antibodies against multiple HPV types, we have generated a recombinant yeast diploid strain that coexpresses the HPV-6 and HPV-16 L1 and L2 genes. HPV-6/16 mosaic VLPs were purified from the cell lysate and used as antigens to immunize mice. The data presented below supports the formation of mosaic VLPs comprising all four proteins. The immunoprecipitation experiment strongly suggests that the CsCl purified VLPs represent the result of a reciprocal interaction of the two L1 proteins, rather than the simple coexistence of different VLP types. The fact that the L2 proteins are present in the same CsCl fractions favors the hypothesis that they are incorporated into the VLPs as well, since the L2 protein alone does not band in a CsCl gradient at the same density as L1 VLPs (22). Further, antisera able to recognize conformational epitopes of both L1 proteins were obtained. Although it remains to be confirmed that the immune response elicited by HPV-6/16 VLPs can neutralize the two viruses, the data herein supports using mosaic VLPs to immunize against a broader spectrum of virus types.

A yeast expression system as herein disclosed is preferred. Different laboratories have observed that a *Saccharomyces cerevisiae* expression system can be successfully used to easily purify PV VLPs (14, 18) which are highly efficient at inducing a protective immune response in animal models (20). Yeast-expressed VLPs are able to elicite a specific immune response not only at systemic, but also at mucosal level. Lowe et al. have reported the generation of IgG neutralizing antibodies in the sera and genital secretions of African green monkeys immunized intramuscularly with HPV-11 VLPs, adsorbed to aluminum adjuvant (27). Greer et al. have observed the induction of anti-L1 specific IgG and IgA antibodies in the sera and genital secretions of mice immunized intranasally with HPV-6 VLPs, adjuvanted either with *E. coli* heat-labile enterotoxin (LT) or with a LT-derived non toxic mutant (14). Further, yeast expression affords the potential to scale-up to thousands of liters at relatively low cost and many yeast-derived products for human use are already market approved due to their safety.

To express the HPV-6 and HPV-16 L1 and L2 genes in the same yeast cell, we generated a *S*. *cerevisiae* diploid strain by mating two haploid strains, each expressing two of the four capsid proteins. In order to obtain expression of the heterologous genes under identical culture conditions, each of them was cloned into the same expression cassette based on the ADH2/GAP glucose-repressible hybrid promoter and the T_{MFα} transcriptional termination sequence. The HPV-6 and HPV-16 L1 proteins were expressed by means of the episomal expression vector pBS24.1. Expression of the HPV-6 and HPV-16 L2 proteins was instead obtained by cloning the expression cassette into an integrative plasmid suitable for insertion into the *lys*2 locus of the haploid strain genome (Fig.4b). As a consequence of this cloning strategy, the L1 and L2 gene copy numbers in the haploid strains were different and this resulted in higher expression levels of the L1 proteins. This should resemble the ratio of L1 to L2 observed in native HPV virions, which has been estimated over a range from 5:1 to 30:1 (25). Table 1 lists the parental yeast strains used, the two recombinant haploid strains obtained and the diploid strain resulting from the mating.

**TABLE 1.**

| List of parental and recombinant yeast strains with genotypes and HPV expressed genes | | | |
|---|---|---|---|
| Yeast strain | Genotype | Episomal HPV gene | Integrated HPVgene |
| JSC310 | *MAT*a *leu*2-3 *ura*3-52 *prb*1-1122 *pep*4-3 *prc*1*-*407 *adr*1::DM15 *cir°* | | |
| AB110 | *MATa leu*2-3-112 *ura*3-52 *pep*4-3 *his*4-580 *cir °* | | |
| JSC310-6L1epi | *MAT*a *prb*1*-*1122 *pep4-3 prc*1*-*407 *adr*1::DM15 *cir °* | 6L1 | |
| JSC310-16L1epi | *MAT*a *prb*1-1122 *pep*4-3 *prc*1-407 *adr*1::DM15 *cir °* | 16L1 | |
| JSC310-6L2epi | *MAT*a *prb*1*-*1122 *pep*4-3 *prc*1-407 *adr*1::DM15 *cir°* | **6L2** | |
| JSC310-16L2epi | *MAT*a *prb*1-1122 *pep*4-3 *prc*1-407 *adr*1::DM15 *cir °* | 16L2 | |
| ISC310-6L2int 6L2 | *MAT*a *leu*2-3 *ura*3-52 *prb*1-1122 *lys*2 *pep*4-3 *prc*1-407 *adr*1 ::DM15 *cir °* | | |
| AB110-16L2int | *MATa leu*2-3-112 *ura*3-52 *pep*4-3 *lys*2 *his*4-580 *cir °* | | 16L2 |
| JSC310-16L1/6L2 6L2 | *MAT*a *prb*1-1122 *lys*2 prc1-407 *pep*4-3 *ade*2 *adr*1::DM15 *cir °* | 16L1 | |
| AB110-6L1/16L2 | *MATa pep*4-3 *lys*2 *his*4-580 *cir °* | 6L1 | 16L2 |
| AB/JSC-4L 16L2 | *MAT*a/*MATa PRB*1/*prb*1-1122 *lys*2/lys2 *PRC*1/*prc*1-407 *pep*4-3/*pep*4-3 | 6L1-16L1 | 6L2- |
| | *HIS*4/*his*4-580 *ADR*1/*adr*1::DM15 *cir °* | | |

As used herein, the term "mosaic VLP" refers to a VLP comprising capsid proteins from more than one type of virus. VLPs which result from intra- and/or inter-capsomeric association of the proteins are included.

As used herein, the term "type" in reference to viruses includes viruses (animal and plant) within the same family, group, or genus as well as viruses in different families, groups, or genuses.

As used herein, the term "non-integrative" in reference to a vector indicates that the vector does not integrate into the host DNA.

**Yeast strains.** The *Saccharomyces cerevisiae* haploid strains used were JSC310 (MATa, *leu*2-3, *ura*3-52*, prb*1-1122, *pep*4-3, *prc*1-407, *adr*1::DM15, *cir°*) (17) and AB 110 (MATα, *leu*2-3-112, *ura*3-52, *pep*4-3, *his*4-580, *cir°*) (43), provided by Vicky Hines (Chiron Corporation, Emeryville, CA, USA). **Monoclonal and polyclonal antibodies.** The H6.C6 and H16.H5 monoclonal antibodies (Mabs), which bind to denatured HPV-6 and HPV-16L1 proteins, respectively, in addition to the H6.B10.5 and H16.V5 Mabs, specific for HPV-6 and HPV-16 intact VLPs, have been reported by Christensen et al. (8, 9). For Western blot analysis, these Mabs were used at 1:3000 dilution with a 4°C overnight incubation. HPV-16 L2 rabbit antiserum was a gift of Lutz Gissmann (DKFZ, Heidelberg, Germany), while HPV-6 L2 rabbit antisera were kindly provided by Denise Galloway (Fred Hutchinson Cancer Research Center Seattle, Washington) and Robert C. Rose (University of Rochester, NY). All the antisera were used at 1:3000-5000 dilution with a 4°C overnight incubation. Anti-rabbit and anti-mouse peroxidase-conjugated antibodies were from Biosource International (Camarillo, CA) and were used at 1:5000 dilution at room temperature for 1.5 hours.

### Example 1

### HPV type-specific detection of capsid proteins expressed in yeast.

A single yeast strain which could express the four HPV-6 and HPV-16 L1 and L2 capsid proteins was prepared. A necessary tool in achieving this was the availability of antibodies which reacted specifically or preferentially with the L1 or the L2 protein of only one HPV type. The HPV-6 and HPV-16 L1 and L2 genes were cloned in the episomal vector pBS24.1 (see Example 2 below) and expressed in the S. *cerevisiae* strain JSC310 to test the type specificity of the available antibodies. Fig. 3 shows the results of a Western blot analysis of total cell extracts prepared from the recombinant strains incubated with specific anti-HPV-6 (a) or HPV-16 (c) L1 Mabs and with HPV-6 (b) or HPV-16 (d) L2 antisera. In all cases HPV type-specific bands were detected, although a weak cross-reactivity could be seen for both the L2 antisera. While the HPV-6 and HPV 16 L1 Mabs identified proteins with the expected molecular weight of about 55 kilodalton (kDa), the L2 proteins, as previously reported (11, 12), showed an electrophoretic mobility corresponding to approximately 72-75 kDa, instead of the 55 kDa predicted on the basis of their amino acid sequences.

### Example 2

### Construction of recombinant plasmids

DNA fragments encoding the HPV proteins were obtained from available recombinant plasmids, either by restriction enzyme digestion or by PCR amplification (Expand High Fidelity PCR System, Boehringer Mannheim), and they were completely sequenced using an Applied Biosystem (Norvalk, CELL TECH, USA) model 373 DNA sequencer.

The episomal yeast expression vector pBS24.1, a yeast "shuttle" vector (17 and Philip J. Barr, Chiron Corporation, Emeryville, CA, USA), containing the leucine 2 (Leu2) and uracil 3 (Ura3) selectable genes was used. In this instance, it was obtained by digesting an available pBS24.1αt6E7 plasmid with Bam HI and Sal I. The pBS24. 1αt6E7 plasmid was prepared for the yeast expression of the HPV-6E7 antigen in a secreted form.

The pBS-6L1 plasmid, expressing the HPV-6 L1 protein under the control of the alcohol-dehydrogenase-2-glyceraldehyde-3-phosphate-dehydrogenase (ADH2/GAP) glucose repressible promoter (J. Shuster, Chiron Corporation, Emeryville, CA, USA) and the mating type alpha factor gene transcriptional termination sequence (T_{MFα}) was derived from the pBS24.1 plasmid as follows.

The plasmid pBS-6L1 is a yeast expression vector which contains the HPV-6L1 under the control of the ADH2\GAP promoter cloned into BAM HI and Sal I sites of the vector pBS24.1. The vector pBS24.1 contains the α-factor terminator, therefore an "expression cassette" for HPV-6 L1 is obtained. The "expression cassette" for HPV-6L1 consists of the following sequences fused together (from 5' to 3'): ADH2\GAP hybrid promoter, HPV-6L1 gene, and α-factor terminator. At the end of the cloning procedures the above "expression cassette" was obtained into the pBS24.1 (17). The vector pBS24.1 may be replicated both in *Escherichia coli* and in *Saccharomyces cerevisiae* since it contains PBR322 sequences (including the origin of replication and the ampicillin resistance gene) and the complete 2µ sequences (including the origin of replication). It also contains the yeast URA3 gene and the yeast LEU2 gene.

A summary of the construction of plasmid pBS24.1-A/G-6L1 is presented schematically in Figure 1. Due to the lack of suitable restriction sites, the fusion between the glucose repressible-ADH2\GAP promoter and the L1 ORF has been obtained by means of recombinant PCR. The 1-563 bp segment of the hybrid promoter (1113 bp long) is derived from GAGαt6E7 plasmid whilst the 564-1113 bp are derived from PCR amplification of Gga plasmid (see below). The 1-115 bp segment of L1 sequence (1503 bp long) is derived from PCR amplification of the pAcC 13-6L 1 plasmid (Greer et al., J., Clin. Microbiology, 2058-2063, 1995 and Munemitsu et al., Mol. Cell. Biol., 10:5977-5982, 1990), whilst the 116-1503 bp segment is derived from pAcC13-6L1 plasmid directly. The DNA sequence of HPV 6 is reported in Schwarz et al., *EMBO J.,* 2:2341-2348, 1983.

The GAGαt6E7 plasmid is a derivative of pGEM-3z (Promega) vector in which the following sequence was constructed (from 5' to 3'): ADH2\GAP promoter, an α-factor derived leader sequence, and the HPV-6E7 coding sequence. The GAGαt6E7 plasmid was digested with Bcl I and Xba I. The DH5α derived plasmid DNA could not be cut with Bcl I because the DH5α cells are dam+, but the Bcl I enzyme is inhibited by overlapping dam methylation; in order to obtain a Bcl I digestible DNA the plasmid was transformed in the dam- JM110 *E. coli* cells (Stratagene). The JM110 derived plasmid was digested with Bcl I and Xba I, the fragment containing the vector and the 5' half of the ADH2\GAP promoter was gel purified and set aside for further ligation.

The pAcC13-6L1 plasmid was digested with Xba I, the insert was gel purified and set aside for ligation. The Xba I insert consisted in the L1 sequence from bp 115 to the end of the sequence, including the stop codon.

The recombinant PCR is schematically represented in Figure 2. The sequences of the primers are listed below.
RP5 5'- ACTGATAGTTTGATCAAAGGGGCAAAACGTAGGGGC-3' SEQ ID NO:1
RP6 5'-GTCGCTAGGCCGCCACATGGTGTTTGTTTATGTGTG-3' SEQ ID NO:2
RP7 5'-AAACACACATAAACAAACACCATGTGGCGGCCTAGC-3' SEQ ID NO:3
RP8 5'-GCAGTCACCACCCTGTACAGGTGTATTAGTACACTG-3' SEQ ID NO:4

A first PCR was performed using the RP5 and RP6 primers and the Gga plasmid DNA as template. The Gga plasmid is a pGEM-3z plasmid derivative obtained in the context of the previous procedures for the HPV-6E7 cloning in yeast and contains the ADH2\GAP promoter. The goal of this first PCR was to obtain the 563-1113 bp portion (3' half) of the ADH2\GAP promoter. The RP5 primer overlapped a Bcl I site. A second PCR was performed using the RP7 and RP8 primers and the pAcC6L1 (Greer et al., 1995) plasmid as template. The goal of this second PCR was to amplify the 5' end of the L1 sequence from the initiation codon to the bp 543. The amplified fragment would contain an Xba I site at position 115. The RP6 and RP7 primers were designed in such a way that the 3' end of the first PCR product would anneal to the 5' end of the second PCR product. A third PCR was performed by mixing the first and second amplimers and the external primers RP5 and RP8. During this PCR a joining between first and second amplimers would happen and also an amplification of the joined product.

The expected 1126 bp product of the third PCR was predicted to consist in the 563-1113 (3' half) sequence of the ADH2\GAP promoter joined to the 1-530 (5' end) sequence of the HPV-6L1 ORF. The final PCR product would have a Bcl I site at the 5' end and an Xba'I site in the L1 portion of the sequence at position 115. The third PCR product was digested with Bcl I and Xba I and gel purified. The fragment containing the pGEM-3z vector and the 5' half of the promoter coming from the Bcl I-Xba I digestion of the GAGαt6E7 plasmid was ligated with the Bcl I-Xba I digested recombinant PCR product and to the L1 insert coming from the Xba I digestion of pAcC13-6L1 plasmid.

After transformation into DH5α cells, several transformants were obtained. The miniprep DNAs from 14 transformants were digested using Eco RI. The Eco RI enzyme was chosen because by using this enzyme it has been possible to verify both the expected molecular sizes and the correct orientation of the 6L 1 fragment. The 6L1 fragment had identical extremities (such as Xba I), therefore the probability for the fragment to assume an opposite orientation was 50%. By using Eco RI the plasmid DNA of the right clones should give two fragments, 2600 and 2700 bp long. The miniprep DNA of the n°8 clone gave a single band on a first gel but by running the gel much more was possible to resolve the 2600 and 2700 bp fragments. Also using Sph I it was possible to have a further indication that the clone n°8 was good. It was, thus, assumed that the clone n°8 contained the correct pGAG-6L1 plasmid consisting in the pGEM-3z vector containing the HPV-6 L1 sequence under the control of the ADH2\GAP promoter.

The ADH2\GAP-HPV-6L1 insert was excised from pGAG-6L1 plasmid by digesting with Barn HI and Sal I, the insert was gel purified and set aside for further ligation. The promoter-L 1 fragment and the pES24.1 vector were ligated and the product of the reaction was transformed into DH5α cells. The miniprep DNAs from 5 transformants were analyzed by digesting the Bam HI and Sal I and the clones A, B, C, and E were selected as good clones exhibiting the right molecular weight pattern.

A clone was transformed in JSC310 strain of *Saccharomyces cerevisiae* by means of electroporation and the cells were plated on URA- plates. Selected transformants were picked from URA- plates and streaked on LEU- plates. Single colonies from LEU -plates were inoculated in LEU- medium. Four clones grown in LEU- medium were reinoculated in YEPD medium. Cell pellets from the four JSC310-6L1 clones, A, B, C and D were frozen at -20°C after 24 and 48 hours of growth in YEPD medium on purpose to check L1 protein expression. Glycerol batches of the four clones were stored at -80°C.

The 6L1 yeast cell pellets were glass beads extracted, soluble and insoluble extracts were separated by means of centrifugation and prepared for SDS-PAGE analysis. Extracts from a strain not containing the pBS-6L1 plasmid (JSC310 cells transformed with pAB24 vector) were also prepared as a negative control. In Coomassie strained gel and in western immunoblot an induced band exhibiting the expected molecular weight was visible. A comparison of the BPV-6L1 expressed in the yeast JSC310 strain and the same antigen expressed in insect cells showed that the two antigens have similar molecular weight.

The DNA portion of the L1 gene deriving from recombinant PCR (bp 1-115) has been sequenced using the following primer:
5' TAGTTTTAAAACACCAA 3' SEQ ID NO: 12.

The primer annealed at the 3' end of the ADH2\GAP promoter, at position -37from the L1 start codon. The pGAG-6L1 plasmid (pGEM-3z containing the ADH2\GAP promoter fused to the L1 sequence) was used as template. By sequencing it was established that no errors occurred during the recombinant PCR manipulations nor in the cloning steps.

To construct the YIpAde integrative plasmid, a 1,059 bp *Xba*I genomic DNA fragment of the *S. cerevisiae* adenine 2 gene (Ade2) was amplified by using the PCR oligonucleotide primers 5'AdeE (5'-GCGGCGAATTCTAGAACAGTTGGTATATTAG-3' SEQ ID NO:5, inserting an *Eco*RI site) and 3'AdeP (5'GCGGCCTGCAGGGTCTAGACTCTTTTCCATATA-3'SEQ ID NO:6, inserting *a Pst*I site). The amplified DNA fragment was cloned into plasmid pUC8 digested with *Eco*RI and *Pst*I and the *Xba*I sites, included in the amplified DNA fragment, were used to excise the insert for yeast transformation. To obtain the integrative YIpLys-L2 expression plasmids, a 1,318 bp genomic DNA fragment of the *S. cerevisiae* lysine 2 (Lys2) gene was amplified by using the PCR oligonucleotide primers 5'LysE (5'-GCGGAATTCCACTAGTAATTACA-3' SEQ ID NO: 7, inserting an *Eco*RI site) and 3'LysH (5'-GATGTAAGCTTCTACTAGTTGA-3'SEQ ID NO:8, inserting a *Hind*III site). The amplified DNA fragment was then inserted into pUC8 (derivatives readily available from commercial sources, e.g., Promega) digested with *Eco*RI and *Hind*III, generating a plasmid named YIpLys. A *Bam*HI DNA fragment from pSI3 vector (Isabel Zaror, Chiron Corporation, Emeryville, CA, USA, pBR322 backbone, ADH2/GAP promoter, SOD protein, and T_{MFα}), including the ADH2/GAP promoter, the human superoxide dismutase (SOD) gene and the T_{MFα} transcriptional termination sequence, was cloned into the single *Bgl*II restriction site in the Lys2 gene sequence of YIpLys, obtaining a plasmid named YIpLys-SOD. The YIpLys-6L2 plasmid was derived from YIpLys-SOD replacing the *Nco*I-*Sal*I DNA fragment encoding the SOD gene with the *Nco*I-*Sal*I DNA fragment from pGEM3z-6L2 (Kent Thudium, Chiron Corporation, Emeryville, CA, USA) encoding the HPV-6b L2 open reading frame (ORF). To construct the YIpLys-16L2 plasmid, the L2 gene was amplified from the cloned HPV-16 genomic DNA (kindly provided in this instance by Dennis J. McCance, University of Rochester, NY) by using the PCR oligonucleotide primers DT-5'L2 (5'-CGACACAAACGTTCTGCAA-3'SEQ ID NO:9) and DT-3'L2 (5'-ATTAGTCGACCTAGGCAGCCAAGAGACATC-3'SEQ ID NO:10), including the translation termination codon and a *Sal*I site. The DNA fragment obtained was digested with *Sal*I and cloned into YIpLys-SOD from which the SOD coding sequence had been removed by digestion with *Nco*I*,* filling-in with Klenow enzyme and digestion with *Sal*I*.*

The pBS-6L2 and pBS-16L2 episomal expression plasmids were obtained by replacing a *Sac*I*-Sal*I DNA fragment from pBS-6L1, including part of the ADH2/GAP promoter and the entire HPV-6b L1 ORF, with *Sac*I-*Sal*I DNA fragments, derived from either YIpLys-6L2 or YIpLys-16L2, including the corresponding promoter region and the L2 ORF.

To construct the pBS-16L1 episomal expression plasmid, the L1 gene was amplified from cloned HPV-16 genomic DNA by using the PCR oligonucleotide primers DT-5'L1 (5'-TCTCTTGGCTGCCTAGTGAGGCCA-3' SEQ ID NO:11) and DT-3'L1 (5'-CTAGTAATGTCGACTTACAGCTTACGTTTGCG-3' SEQ ID NO: 12), comprising the translational termination codon and a *Sal*I site. The amplified DNA fragment was purified from agarose gel and cloned into blunt-ended pSI3 vector from which the SOD gene had been previously removed by digestion with *Nco*I and *Sal*I restriction enzymes and filling-in with Klenow enzyme. From this intermediate construct, a *Sac*I*-Sal*I DNA fragment, including part of the ADH2/GAP promoter and the HPV-16L1 ORF, was purified and used to replace the corresponding *Sac*I*-Sal*I DNA fragment in pBS-6L1.

### Example 3

### Generation of recombinant yeast strains

The strains JSC310-6L1epi (14), JSC310-16L1epi, JSC310-6L2epi and JSC310-16L2epi, expressing the four capsid proteins by means of episomal vectors, were obtained by transformation of the parental JSC310 strain with the expression plasmids pBS-6L1 (14), pBS-16L1, pBS-6L2 and pBS-16L2.

The JSC310-6L2int and the AB 110-16L2int strains were obtained using the following experimental approach. Competent yeast cells were cotransformed with 5µg of *Eco*RI-*Hind*III digested YIpLys-6L2 or YIpLys-16L2 integrative plasmid and 1 µg of pBS24.1 episomal vector to allow the selection of transformants. Different clones were tested for growth onto plates of minimal medium (MM) supplemented with α-adipate to select mutants with an inactivated Lys2 gene (49). Correct integration into the *lys*2 locus was verified by PCR analysis by using pairs of oligonucleotide primers complementary to sequences within the expression cassette and the genomic portion of the Lys2 gene. Among the colonies expressing the L2 protein, one was chosen, cured of the pBS24.1 plasmid and tested for the inability to grow in the absence of uracil and leucine. Introduction of the episomal L1 expressing vectors into these strains was carried out following two different strategies. AB 110-16L2int was transformed with the pBS-6L1 expression plasmid and selection of transformants on MM plates without leucine and uracil allowed the isolation of the haploid strain AB 110-6L1/16L2. The JSC310-6L2int strain was instead cotransformed with the pBS-16L1 expression vector and with the *Xba*I digested YIpAde integrative plasmid. Transformants grown on selective plates were plated on complete yeast extract-peptone medium (YEP) and allowed to grow at 30°C for 3-4 days until colonies (1-2%) developed a red color due to disruption of the *ade*2 locus (52). One of the clones, which showed correct integration into the *ade*2 locus by PCR and L1 and L2 expression by Western blot analysis, was designated JSC310-16L 1/6L2.

Generation of the AB/JSC-4L diploid strain was obtained by mixing cultures, in YEP medium containing 5% glucose, of the two haploid strains, AB 110-6L1/16L2 and JSC310-16L1/6L2. Selection of the AB/JSC-4L diploid strain required an additional genetic marker in the haploid JSC310-6L2int strain. This was obtained inactivating the endogenous Ade2 gene by means of the integration plasmid represented in Fig.4a. Diploid cells were selected onto MM plates lacking histidine and adenine.

Expression of the four proteins in the haploid strains and in the strain resulting from their mating was evaluated by Western blot analysis. Fig. 5 shows the results of such experiments demonstrating that both the haploid strains AB110-6L1/16L2 (a and d, lanes 1) and JSC310-16L1/6L2 (b and c, lanes 2) expressed the heterologous genes and that the expression of all four proteins was stably maintained in the resulting AB/JS-4L diploid strain (a, b, c and d, lanes 3).

### Example 4

### Preparation of VLPs

Parental yeast strains were grown in complete YEP medium. Strains transformed with episomal vectors were first cultured in leucine-deficient MM medium with 4% glucose until they reached midlog phase. Expression of the genes under the control of the ADH2/GAP glucose-repressible promoter was induced by diluting these cultures 1:50 into YEP complete medium and culturing the cells at 30°C for 2-3 days. Total cell extracts were prepared from 3.5 optical densities (OD) of yeast cell cultures grown to approximately OD₆₀₀=20. Cells were lysed with a 10 minute incubation on ice in 0.24 N NaOH and 0.96% β-mercaptoethanol, followed by trichloroacetic acid (TCA) precipitation, ice cold acetone washing and final suspension of the protein pellet in 100 µl of protein loading buffer. To carry out dot-blot experiments where preservation of L1 conformation was necessary, yeast cells were collected, washed, suspended in phosphate-buffered saline (PBS, pH 7.5) and disrupted by vortexing five times for 1 minute in the presence of glass beads (425-600 µm, Sigma).

Frozen yeast cell pellets were thawed in buffer containing 0.1 M Tris-HCl (pH 7.5),0.15 M NaCl, 2 mM MgCl₂ and 1 mM EGTA (#E3889, Sigma Chemical Co.) and Complete™Protease Inhibitors (#1-697-498, Boehringer Mannheim). Cells were disrupted by vortexing twice for 10 minutes, with a 5 minute interval on ice, in the presence of glass beads (0.5 ml beads per ml of cell suspension) using a VWRbrand Multi-tube vortexer (VWR Scientific Product). Cellular debris was removed by a 20 minute centrifugation at 2000 x g. The supernatants were then centrifuged through a 40% (w/w) sucrose cushion (2 hour centrifugation at 100,000 x g). The resulting pellets were suspended in PBS, applied to a pre-formed CsCl gradient (1.17-1.57 g/ml) and centrifuged for 24 hours at 285,000 x g. The gradients were fractionated and aliquots from each fraction were subjected to Western blot analysis with type-specific anti-L1 and-anti-L2 antibodies. Peak fractions were pooled and dialyzed against PBS. Total protein concentration was determined by BCA^{TM} Protein Assay Reagent (#23225, Pierce Chemicals).

### Example 5

### Characterization of VLPs

Proteins were analyzed by denaturing sodium-dodecyl sulfate polyacrylamide gel electrophoresis (SDS-PAGE, 10% polyacrylamide) and Western blotting onto nitrocellulose membrane (pore size 0.45 µm, MSI, Westborough, MA USA) according to standard protocols. Dot-blot analysis of denatured and reduced VLPs was carried out boiling the protein samples for 5 minutes in the presence of dithiothreitol (DTT) before applying them to nitrocellulose filters using a bio-dot apparatus (Biorad). When native VLP structure had to be maintained, VLPs in PBS were applied to the membrane without boiling and in the absence of DTT. Reaction with HPV-specific antibodies was detected using the Enhanced Chemiluminescence (ECL) Western blotting reagent (Amersham) and Hyperfilm ECL (Amersham).

Specifically, the cell extract from the diploid strain was subjected to CsCl gradient sedimentation and aliquots of the collected fractions were boiled in the presence of DTT and blotted in duplicate onto nitrocellulose filters. The filters were incubated with anti-HPV-6 and anti-HPV-16 specific Mabs which react with denatured L1 (8, 9), revealing that the two L1 proteins were enriched in the same fractions (Fig. 6A, a and c). The dot-blot experiment was repeated without denaturing and without reducing the protein samples and using anti-HPV-6 and HPV-16 L1 specific Mabs which were previously reported to react exclusively with intact VLPs in enzyme-linked immunosorbent assay (ELISA) experiments (8, 9). The result obtained confirmed that the two conformationally dependent Mabs were able to recognize the L1 proteins which copurified in the CsCl fractions (Fig 6A, b and d). As expected, the two Mabs reacted specifically with HPV-6 and HPV-16 control VLPs only under nondenaturing and nonreducing conditions (Fig. 6A, e). Western blot analysis of fraction 5 confirmed that both HPV-6 and HPV-16 L2 proteins were also present (Fig. 6B, a and b). Estimation of the refractive index of the identified protein peak gave a value of 1.29-1.3 mg/ml. EM analysis of the enriched fraction revealed the presence of VLPs which appeared to be similar to control VLPs formed by either HPV-6 or HPV-16 L1 (Fig.7).

To evaluate whether the HPV-6 and HPV 16 L1 proteins could interact and assemble into mosaic VLPs, we performed immunoprecipitation experiments using CsCl banded VLPs and the specific anti-HPV-6 L1 conformationally dependent Mab H6.B10.5 (9). Approximately 1 µg of CsCL banded VLPs were diluted with PBS and incubated with the conformationally dependent anti-HPV-6 L1 Mab H6.B 10.5 (1:1000 dilution) overnight at 4° C with gentle shaking. The immune complexes were collected with Protein A Sepharose CL-4B (Pharmacia Biotech), washed 4 times with 1 ml PBS, suspended in sample buffer, boiled for 5 minutes, subjected to SDS-PAGE and analyzed by Western blot using anti-HPV-6 and anti-HPV-16 L1 Mabs. The Western blot carried out on the immunoprecipitates using type-specific anti-L1 Mabs (Fig. 8) identified three major bands: (A) was a Mab-derived band, since it could be also observed when the conformational Mab was immunoblotted with the anti-mouse antibody; (B) was a band that appeared only when the VLPs were incubated with the conformational anti-HPV-6 L1 Mab (lanes 3), identifying specifically immunoprecipitated proteins with an electrophoretic mobility corresponding to that of HPV-6 L1 (a, lane 4) and HPV-16 L1 (b, lane 5); (C) was a resin-derived band that was also detected when an aliquot of protein A Sepharose was suspended in PBS and immunoblotted with the anti-mouse antibody. Bands (B) were not visible when the immunoprecipitation was carried out using an unrelated Mab Similarly, HPV-16 L1 could not be detected when HPV-6 and HPV-16 VLPs were mixed and immunoprecipitated.

### Example 6

### Mouse immunization with VLPs.

To investigate whether HPV-6/16 mosaic VLPs were able to induce an immune response directed against both HPV types, groups of mice were immunized subcutaneously with HPV-6, HPV-16 and mosaic VLPs and the sera were tested after the third immunization. Six week old female Balb/c mice were injected subcutaneously with 20 µg of the following purified antigens: (I) HPV-6 VLPs, (ii) HPV-16 VLPs, (iii) HPV-6/16 VLPs. All the antigens were administered with equal volume of MF59 adjuvant (30). A group of control mice was injected only with MF59. The mice were boosted with 15 µg of the respective antigen at week 3 and 10 µg at week 5. Serum samples were collected on day 12 after the final booster and assayed for capsid protein specific antibodies.

Figure 9A shows the result of the Western blot carried out with the three types of denatured VLPs incubated with three sera, each representative of the different groups of immunized mice. While the reactivity of the sera from mice immunized either with HPV-6 or HPV-16 VLPs was predominantly type-specific (Fig. 9A, a and b), the serum from mouse 16 (S16), immunized with HPV6/16 VLPs, reacted against both HPV-6 and HPV-16 L1 (Fig. 9A, c). To analyze whether the immune response was also directed against conformational epitopes of the L1 proteins, equal amounts of either HPV-6 or HPV-16 VLPs were blotted under denaturing and nondenaturing conditions and incubated with the S16 antiserum. Figure 9B shows that the signal was significantly lower when the samples were denatured and reduced, suggesting that conformational antibodies had been elicited.

The foregoing examples are meant to illustrate the invention and are not to be construed to limit the invention in any way. Those skilled in the art will recognize modifications that are within the spirit and scope of the invention. All references cited herein are hereby incorporated by reference in their entirety.

### REFERENCES

1. **Bonnez, W., R. C. Rose, C. da Rin, C. Borkhuis, K. de Mesy Jensen, and R. C. Reichman.** 1993. Propagation of human papillomavirus type 11 in human xenografts using the severe combined immunodeficiency (SCID) mouse and comparison to the nude mouse model. Virology **197**:455-458.
2. **Bonnez, W., C. da Rin, C. Borkhuis, K. de Mesy Jensen, R. C. Reichman, and R. C. Rose.** 1998. Isolation and propagation of human papillomavirus type 16 in human xenografts implanted in the severe combined immunodeficiency mouse. J. Virol. **72**:5256-5261.
3. **Chan, S. Y., H. U. Bernard, C. K. Hong, S. P. Chan, B. Hoffmann, and H. Delius.** 1992. Phylogenetic analysis of 48 papillomavirus types and 28 subtypes and variants: a showcase for the molecular evolution of DNA viruses. J. Virol. 66:5714-5725.
4. **Chang, C., S. Zhou, D. Ganem, and D. N. Standring.** 1994. Phenotypic mixing between different hepadnavirus nucleocapsid proteins reveals C protein dimerization to be *cis* preferential. J. Virol. **68:**5225-5231.
5. **Christensen, N. D., and J. W. Kreider.** 1990. Antibody-mediated neutralization in vivo of infectious papillomavirus. J. Virol. **64**:3151-3156.
6. **Christensen, N. D., J. W. Kreider, N. M. Cladel, S. D. Patrick, and P. A. Welsh.** 1990. Monoclonal antibody-mediated neutralization of infectious human papillomavirus type 11. J. Virol. **64**:5678-5681.
7. **Christensen, N. D., R. Kirnbauer, J. T. Schiller, S. J. Ghim, R. Schlegel, and J. W. Kreider.** 1994. Human papillomavirus types 6 and 11 have antigenically distinct strongly immunogenic conformationally dependent neutralizing epitopes. Virology **205**:329-335.
8. **Christensen, N. D., C. A. Reed, N. M. Cladel, K. Hall, and G. S. Leiserowitz.** 1996. Monoclonal antibodies to HPV-6 L1 virus-like particles identify conformational and linear neutralizing epitopes on HPV 11 in addition to type-specific epitopes on HPV-6. Virology **224**:477-486.
9. **Christensen, N. D., J. Dillner, C. Eklund, J. J. Carter, G. C. Wipf, C. A. Reed, N. M. Cladel, and D. A. Galloway.** 1996. Surface conformational and linear epitopes on HPV-16 and HPV 18 L1 virus-like particles as defined by monoclonal antibodies. Virology **223**:174-184.
10. **Deminie, C. A., and M. Emerman.** 1993. Incorporation of human immunodeficiency virus type 1 Gag proteins into murine leukemia virus virions. J. Virol. **67**:6499-6506. 11. **Doorbar, J., and P. H. Gallimore.** 1987. Identification of proteins encoded by the L1 and L2 open reading frames of human papillomavirus 1a. J. Virol. **61**:1131-1142.
12. **Firzlaff, J. M., N. B. Kiviat, A. M. Beckmann, S. A. Jenison, and D. A. Galloway.** 1988. Detection of human papillomavirus capsid antigens in various squamous epithelial lesions using antibodies directed against the L1 and L2 open reading frames. Virology **164**:467-477.
13. **Franke, E. K., H. E. H. Yuan, K. L. Bossolt, S. P. Goff, and J. Luban.** 1994. Specificity and sequence requirements for interactions between various retroviral Gag proteins. J. Virol. **68**:5300-5305.
14. **Greer K. E., R. Petracca, B. Gervase, D. Tornese Buonamassa, M. Ugozzoli, A. Di Tommaso, M. T. De Magistris, G. Van Nest, and G. Bensi.** in preparation.
15. **Hagensee, M. E., N. Yaegashi, and D. A. Galloway.** 1993. Self-assembly of human papillomavirus type 1 capsids by expression of L1 protein alone or by coexpression of the L1 and L2 capsid proteins. J. Virol. **67**:315-322.
16. **Hagensee, M. E., N. H. Olson, T. S. Baker, and D. A. Galloway.** 1994. Three-dimensional structure of vaccinia virus-produced human papillomavirus type 1 capsids. J. Virol. **68**:4503-4505.
**17. Hines, V., W. Zhang, N. Ramakrishna, J. Styles, P. Mehta, K. S. Kim, M. Innis, and D. L. Miller.** 1994. The expression and processing of human beta-amyloid peptide precursors in *Saccharomyces cerevisiae:* evidence for a novel endopeptidase in the yeast secretory system. Cell. Mol. Biol. Res. **40**:273-284.
18. **Hofmann, K. J., J. C. Cook, J. G. Joyce, D. R. Brown, L. D. Schultz, H. A. George, M. Rosolowsky, K. H. Fife, and K. U. Jansen.** 1995. Sequence determination of human papillomavirus 6a and assembly of virus-like particles in *Saccharomyces cerevisiae.* Virology **209**:506-518.
**19. Hofmann, K. J., M. P. Neeper, H. Z. Markus, D. R Brown, M. Muller, and K. U. Jansen.** 1996. Sequence conservation within the major capsid protein of human papillomavirus (HPV) type 18 and formation of HPV-18 virus-like particles in *Saccharomyces cerevisiae.* J. Gen. Virol. **77**:465-468.
20. **Jansen, K. U., M. Rosolowsky, L. D. Schultz, H. Z. Markus, J. C. Cook, J. J. Donnelly, D. Martinez, R. W. Ellis, and A. R. Shaw.** 1995. Vaccination with yeast-expressed cottontail rabbit papillomavirus (CRPV) virus-like particles protects rabbits from CRPV-induced papilloma formation. Vaccine **13**:1509-1514.
21. **Kirnbauer, R., G. Booy, N. Cheng, R. R. Lowy, and J. T. Schiller.** 1992. Papillomavirus L1 major capsid protein self-assembles into virus-like particles that are highly immunogenic. Proc. Natl. Acad. Sci. USA **89**:12180-12184.
22. **Kirnbauer, R**.**, J. Taub, H. Greenstone, R. B. S. Roden, M. Durst, L. Gissmann, D. R. Lowy, and J. T. Schiller.** 1993. Efficient self-assembly of human papillomavirus type 16 L1 and L1-L2 into virus-like particles. J. Virol. **67**:6929-6936.
23. **Kirnbauer, R., L. M. Chandrachud, B. W. O'Neil, E. R. Wagner, G. J. Grindlay, A. Armstrong, G. M. McGarvie, J**. **T. Schiller, D. R. Lowy, and M. S. Campo.** 1996. Virus-like particles of bovine papillomavirus type 4 in prophylactic and therapeutic immunization. Virology **219**:37-44.
**24. Kreider, J. W., M. K. Howett, A. E. -Leure-Dupree, R. J. Zaino, and J. A. Weber.** 1987. Laboratory production in vivo of infectious human papillomavirus type 11. J. Virol. **61**:590-593.
25. **Li, M., T. P. Cripe, P. A. Estes, M. K. Lyon, R. C. Rose, and R. L. Garcea.** 1997. Expression of the human papillomavirus type-11 capsid protein in *Escherichia coli:* characterization of protein domains involved in DNA-binding and capsid assembly. J. Virol. **71**:2988-2995.
**26. Li, M., P. Beard, P. A. Estes, M. K. Lyon, and R. L. Garcea.** 1998. Intercapsomeric disulfide bonds in papillomavirus assembly and disassembly. J. Virol. **72**:2160-2167.
**27. Lowe, R. S., D. R. Brown, J. T. Bryan, J. C. Cook, H. A. George, K. J. Hofmann, W. M. Hurni, J. G. Joyce, E. D. Lehman, H. Z. Markus, M. P. Neeper, L. D. Schultz, A. R. Shaw, and K U. Jansen.** 1997. Human papillomavirus type 11 (HPV-11) neutralizing antibodies in the serum and genital mucosal secretions of African green monkeys immunized with HPV-11 virus-like particles expressed in yeast. J. Infect. Dis. **176**:1141-1145.
**28. Muller, M., L. Gissmann, R. J. Cristiano, X. Y. Sun, H. Frazer, A. B. Jenson, A. Alonso, H. Zentgraf, and J. Zhou.** 1995. Papillomavirus capsid binding and uptake by cells from different tissues and species. J. Virol. **69**:948-954.
29. **Neeper, M. P., K. J. Hofmann, and K. U. Jansen.** 1996. Expression of the major capsid protein of human papillomavirus type 11 in *Saccharomyces cerevisiae.* Gene **180**:1-6.
30. **Ott, G., G. L. Barchfeld, D. Chernoff, R. Radakrishnan, P. van Hoogevest and G. van Nest.** 1995. MF59: Design and evaluation of a safe and potent adjuvant for human vaccines, p. 277-296. *In* M. F. Powell and M. J. Newman (ed.), Vaccine design. The subunit and adjuvant approach. Plenum Press, New York, N. Y.
31. **Qi, Y. M., S. W. Peng, K. Hengst, M. Evander, D. S. Park, J. Zhou, and I. A. Frazer.** 1996. Epithelial cells display separate receptors for papillomavirus VLPs and for soluble L1 capsid protein. Virology 216:3.5-45.
32. **Roden, R. B., E. M. Weissinger, D. W. Henderson, F. Booy, R. Kirnbauer, J. F. Mushinski, D. R. Lowy and J. T. Schiller.** 1994. Neutralization of bovine papillomavirus by antibodies to **L1** and L2 capsid proteins. J. Virol. **68**:7570-7574
33. **Roden, R. B., R. Kirnbauer, A. B. Jenson, D. R. Lowy, and J. T. Schiller.** 1994. Interaction of papillomaviruses with the cell surface. J. Virol. **68**:7260-7266.
34. **Roden, R. B. S., H. L. Greenstone, R. Kirnbauer, F. P. Booy, J. Jessie, D. R. Lowy, and J. T. Schiller.** 1996. *In vitro* generation and type-specific neutralization of human papillomavirus type 16 virion pseudotype. J. Virol. **70**:5875-5883.
35. **Roden, R. B. S., N. L. Hubbert, R. Kirnbauer, N. D. Christensen, D. R. Lowy, and J. T. Schiller.** 1996. Assessment of serological relatedness of genital human papillomaviruses by hemagglutination inhibition. J. Virol. **70**:3298-3301.
36. **Rose, R. C., W. Bonnez, C. da Rin, D. J. McCance, and R. C. Reichman.** 1994. Serological differentiation of human papillomavirus types 11, 16 and 18 using recombinant virus-like particles. J. Gen. Virol. **75**:2445-2449.
37. **Rose, R. C., W. Bonnez, R. C. Reichman, and R. L. Garcea.** 1993. Expression of human papillomavirus type 11 L1 protein in insects cells: *in vivo* and *in vitro* assembly of virus-like particles. J. Virol. **67**:1936-1944.
38. **Sapp, M., C. Volpers, M. Muller, and RE. Streeck.** 1995. Organization of the major and minor capsid proteins in human papillomavirus type 33 virus-like particles. J. Gen. Virol. **76**:2407-2412.
39. **Sapp, M., C. Fligge, I. Petzak, J. R. Harris, and R. E. Streeck.** 1998. Papillomavirus assembly requires trimerization of the major capsid protein by disulfides between two highly conserved cysteines. J. Virol. **72**:6186-6189.
40. **Smith, L. H., C. Foster, M. E. Hitchcock, G. S. Leiserowitz, K. Hall, R. Iseroff, N. D. Christensen, and J. W. Kreider.** 1995. Titration of HPV-11 infectivity and antibody neutralization can be measured *in vitro.* J. Invest. Dermatol. **105**:1-7.
41. **Suzich, J. A., S. Ghim, F. J. Palmer-Hill, W. I. White, J. K. Tamura, J. A. Bell, J. A. Newsome, A. Bennet Jenson, and R. Schlegel.** 1995. Systemic immunization with papillomavirus **L1** protein completely prevents the development of viral mucosal papillomas. Proc. Natl. Acad. Sci. USA **92**:1553-11557.
42. **Touzé, A., C. Dupuy, D. Mahé, P. Y. Sizaret, and P. Coursaget.** 1998. Production of recombinant virus-like particles from human papillomavirus type 6 and 11, and study of serological reactivities between HPV 6, 11 and 45 by ELISA: implications for papillomavirus prevention and detection. FEMS Microbiol. **160**: 111-118.
43. **Travis, J., M. Owen, P. George, R. Carrel, S. Rosenberg, R. A. Hallewell, and P. J. Barr.** 1985. Isolation and properties of recombinant DNA produced variants of human α₁-proteinase inhibitor. J. Biol. Chem. **260**:4384-4389.
44. **Unckell, F., R. E. Streeck, and M. Sapp.** 1997. Generation and neutralization of pseudovirions of human papillomavirus type 33. J. Virol. **71**:2934-2939.
45. **Van Ranst, M., J. B. Kaplan, and R. D. Burk.** 1992. Phylogenetic classification of human papillomaviruses: correlation with clinical manifestations. J. Gen. Virol. **73**:2653-2660.
46. **Volpers, C., P. Schirmacher, R. E. Streeck, and M. Sapp.** 1994. Assembly of the major and the minor capsid protein of human papillomavirus type 33 into virus-like particles and tubular structures in insect cells. Virology **200**: 504-512.
**47. Volpers, C., F. Unckell, P. Schirmacher, R. E. Streeck, and M. Sapp.** 1995. Binding and internalization of human papillomavirus type 33 virus-like particles by eukaryotic cells. J. Virol. **69**:3258-3264.
**48. White, W. I., S. D. Wilson, W. Bonnez, R. C. Rose, S. Koenig, and J. A. Suzich.** 1998. *In vitro* infection of type-restricted antibody-mediated neutralization of authentic human papillomavirus type 16. J. Virol. **72**:959-964.
49. **Zaret, K. S., and F. Sherman.** 1985. α-Aminoadipate as a primary nitrogen source for *Saccharomyces cerevisiae* mutants. J. Bacteriol. **162**:579-583.
50. **Zàvada, J.** 1982. The pseudotypic paradox. J. Gen. Virol. **63**:15-24.
51. **Zhou, J., D. J. Stenzel, X. Y. Sun, and I. H. Frazer.** 1993. Synthesis and assembly of infectious bovine papillomavirus particles *in vitro.* J. Gen. Virol. **74**:763-768.
52. Zimmerman, F.K. 1975. Procedures used in the induction of mitotic recombination and mutation in the yeast *Saccharomyces cerevisiae.* Mutat. Res. **31**:71-86.
53. Synthetic HPV 16 virus-like particles. WO 98/14564
54. Molecular presenting system. WO 96/05293

## Claims

1. A method for producing VLPs comprising capsid proteins from at least HPV type 6 and HPV type 16, said method comprising:
a) cloning said capsid proteins into expression cassettes comprising the same promoters and termination signals;
b) expressing said cassettes in the same host cell;
wherein L1 protein expression cassettes are cloned into non-integrative vectors and L2 protein expression cassettes are cloned into integrative vectors.

2. A method for expressing capsid proteins from at least HPV type 6 and HPV type 16, said method comprising:
a) cloning said capsid proteins into expression cassettes comprising the same promoters and termination signals;
b) expressing said cassettes in the same host cell;
wherein L1 protein expression cassettes are cloned into non-integrative vectors and L2 protein expression cassettes are cloned into integrative vectors.

3. The method according to claim 1 or claim 2, wherein the host cell is a yeast cell.

4. The method according to claim 3, wherein the yeast cell is *Saccharomyces cerevisiae.*

5. The method according to any previous claim, wherein the non-integrative vector is pBS24.1.

6. The method according to any previous claim, wherein the integrative vector is pUC8.

## Patentansprüche

1. Verfahren zur Herstellung von Virus-ähnlichen Partikeln (VLPs), die Kapsidproteine von mindestens dem HPV-Typ 6 und dem HPV-Typ 16 enthalten, wobei das Verfahren umfasst:
a) Einklonieren dieser Kapsidproteine in Expressionskassetten,
welche die gleichen Promotoren und die gleichen Terminationssignale aufweisen,
und
b) Exprimieren der Kassetten in der gleichen Wirtszelle, wobei die L1-Protein-Expressionskassetten in nicht-integrative Vektoren und die L2-Protein-Expressionskassetten in integrative Vektoren einkloniert werden.

2. Verfahren zur Expression von Kapsid-Proteinen von mindestens dem HPV-Typ 6 und dem HPV-Typ 16, wobei das Verfahren umfasst:
a) Einklonieren dieser Kapsidproteine in Expressionskassetten,
welche die gleichen Promotoren und die gleichen Terminationssignale aufweisen,
und
b) Exprimieren der Kassetten in der gleichen Wirtszelle, wobei die L1-Protein-Expressionskassetten in nicht-integrative Vektoren und die L2-Protein-Expressionskassetten in integrative Vektoren einkloniert werden.

3. Verfahren nach Anspruch 1 oder 2, wobei die Wirtszelle eine Hefezelle ist.

4. Verfahren nach Anspruch 3, wobei die Hefezelle eine Zelle von *Saccharomyces cerevisiae* ist.

5. Verfahren nach einem der vorhergehenden Ansprüche, wobei der nicht-integrative Vektor pBS24.1 ist.

6. Verfahren nach einem der vorhergehenden Ansprüche, wobei der integrative Vektor pUC8 ist.

## Revendications

1. Procédé de production de particules de type virus (VLP) comprenant des protéines de capside provenant au moins du papillomavirus humain (HPV) de type 6 et du HPV de type 16, ledit procédé comprenant les étapes consistant à :
a) cloner lesdites protéines de capside dans des cassettes d'expression, comprenant les mêmes promoteurs et signaux de terminaison, et
b) exprimer lesdites cassettes dans la même cellule hôte ;
dans lequel les cassettes d'expression de la protéine L1 sont clonées dans des vecteurs non intégrants, et les cassettes d'expression de la protéine L2 sont clonées dans des vecteurs intégrants.

2. Procédé d'expression de protéines de capsides provenant au moins du papillomavirus humain (HPV) de type 6 et du HPV de type 16, ledit procédé comprenant les étapes consistant à :
a) cloner lesdites protéines de capside dans des cassettes d'expression, comprenant les mêmes promoteurs et signaux de terminaison, et
b) exprimer lesdites cassettes dans la même cellule hôte ;
dans lequel les cassettes d'expression de la protéine L1 sont clonées dans des vecteurs non intégrants, et les cassettes d'expression de la protéine L2 sont clonées dans des vecteurs intégrants.

3. Procédé selon la revendication 1 ou la revendication 2, dans lequel la cellule hôte est une cellule de levure.

4. Procédé selon la revendication 3, dans lequel la cellule de levure est une cellule de *Saccharomyces cerevisiae.*

5. Procédé selon l'une des revendications précédentes, dans lequel le vecteur non intégrant est pBS24 .1.

6. Procédé selon l'une des revendications précédentes, dans lequel le vecteur intégrant est pUC8.
